Europäisches Patentamt

European Patent Office

Office européen des brevets

(19)

(11) Veröffentlichungsnummer: **0 076 363**

**A1**

(12) # EUROPÄISCHE PATENTANMELDUNG

(21) Anmeldenummer: 82106037.3

(22) Anmeldetag: 06.07.82

(51) Int. Cl.³: **A 61 N 1/36**

(30) Priorität: 13.07.81 DE 3127597

(43) Veröffentlichungstag der Anmeldung:
13.04.83 Patentblatt 83/15

(84) Benannte Vertragsstaaten:
DE FR GB NL SE

(71) Anmelder: Siemens-Elema AB
Röntgenvägen 2
S-171 95 Solna 1(SE)

(84) Benannte Vertragsstaaten:
SE

(71) Anmelder: SIEMENS AKTIENGESELLSCHAFT
Berlin und München Wittelsbacherplatz 2
D-8000 München 2(DE)

(84) Benannte Vertragsstaaten:
DE FR GB NL

(72) Erfinder: Elmqvist, Hakan, Dr.
Sunnerdalsvägen 7
S-16 138 Bromma(SE)

(54) Herzschrittmacher zur bifokalen Stimulierung des Herzens.

(57) Bei einem Herzschrittmacher zur bifokalen Stimulierung des Herzens werden mittels eines Impulsgenerators Stimulierungsimpulse für die Vorkammer und zeitverzögert für die Herzkammer geliefert. Über einen Detektor werden dabei die spontanen Aktivitäten der Vorkammer erfasst und zur Steuerung des Impulsgenerators benutzt. Um die hämodynamisch effektive PQ-Zeit optimal den Aktivitäten in der Vorkammer anzupassen, ist erfindungsgemäss vorgesehen, dass die elektrische Verzögerungszeit (AV-Zeit) zwischen einer Vorkammeraktivität und dem Stimulierungsimpuls für die Herzkammer zwei Werte (AV-I, AV-II) annimmt, je nachdem, ob es sich um eine spontane Vorkammeraktivität oder einen Stimulierungsimpuls handelt. Der Herzschrittmacher enthält ein Verzögerungsglied (4), das den Stimulierungsimpuls für die Herzkammer nach unterschiedlichen Zeiten freigibt.

FIG 1

EP 0 076 363 A1

SIEMENS AKTIENGESELLSCHAFT      Unser Zeichen
Berlin und München              VPA 81 P 5262 E

Herzschrittmacher zur bifokalen Stimulierung des
Herzens

Die Erfindung betrifft einen Herzschrittmacher zur bifokalen Stimulierung des Herzens mittels eines Impulsgenerators, der Stimulierungsimpulse für die Vorkammer
und zeitverzögert für die Herzkammer liefert und der
über mindestens einen Detektor die spontanen Aktivitäten der Vorkammer erfasst und zur Steuerung des
Impulsgenerators benutzt.

Bei einem bekannten Herzschrittmacher wird die Aktivität der Vorkammer (Atrium) durch einen Detektor überwacht. Beim Ausbleiben einer spontanen P-Welle wird
ein Stimulierungsimpuls an die Vorkammer abgegeben.
Unabhängig von der Art der Vorkammeraktivität wird
anschliessend nach einer festen Verzögerungszeit ein
Impuls für die Herzkammer (Ventrikel) erzeugt. Dabei
ist es durch einen weiteren Detektor möglich, auch
die spontane Aktivität der Herzkammer zu überwachen
und nur beim Ausbleiben einer derartigen Aktivität
den Stimulierungsimpuls an die Herzkammer weiterzuleiten.

Der Erfindung liegt die Aufgabe zugrunde, einen Herzschrittmacher der eingangs genannten Art derart zu
verbessern, dass die Herztätigkeit stärker einem natürlichen Verlauf angepasst und damit das physische
Empfinden eines Patienten positiv beeinflust wird.
Weiterhin liegt der Erfindung die Aufgabe zugrunde,
einen Herzschrittmacher anzugeben, mit dem diese Anpassung auf einfache und sichere Weise realisiert
werden kann. Die erste Aufgabe wird dadurch gelöst, dass die Zeitverzögerung in Abhängigkeit von den Aktivitäten in der Vorkammer unterschiedlich lang gewählt wird. Dabei stützt sich die Erfindung auf die gewonnene Erkenntnis, dass die hämodynamisch effektive PQ-Zeit, d.h. die Zeit zwischen einer Vorkammerkontraktion (P-Zacke)·und der darauffolgenden Kammerkontraktion (QRS-Komplex), bei Verwendung einer einzigen konstanten Verzögerungszeit ungleich lang ist, je nachdem ob die elektrische AV-Zeit, d.h. die Zeit zwischen einem ersten Ereignis in der Vorkammer (Atrium), sei es nun Stimulierungsimpuls oder spontane P-Zacke, und dem darauffolgenden Stimulierungsimpuls für die Herzkammer (Ventrikel), durch eine spontane Vorkammer kontraktion (P-Zacke) oder durch einen Stimulierungsimpuls in der Vorkammer ausgelöst wird. Einem Stimulierungsimpuls folgt nämlich erst nach einer Verzögerung zwischen ca: 20-150 Millisekunden die P-Zacke, der dann in einem sehr viel kürzeren Abstand als bei einer spontanen P-Zacke der durch den Stimulilerungsimpuls für die Herzkammer ausgelöste QRS-Komplex folgt. Diese unterschiedlichen PQ-Zeiten können zu einer Beeinträchtigung des Wohlbefindens eines Patienten führen.

Im Gegensatz dazu sieht der erfindungsgemässe Herzschrittmacher vor, dass die elektrische AV-Zeit zwei unterschiedliche Werte annimmt. Wird die PQ-Zeit durch eine spontane Vorkammerdepolarisation angeregt, d.h. wird eine spontane P-Zacke durch den Detektor erfasst, so folgt die Stimulierung der Herzkammer mit einer ersten Zeitverzögerung AV-I. Wird hingegen die PQ-Zeit durch einen Stimulierungsimpuls in der Vorkammer ausgelöst, so erfolgt die Stimulierung der Herzkammer nach einer anderen Zeitverzögerung AV-II. In Weiterbildung der Erfindung ist dabei vorgesehen, dass AV-II länger als AV-I ist.

Um den Herzschrittmacherweiterhin noch besser an unterschiedliche Bedingungen, insbesondere unterschiedliche Patienten, anpassen zu können, ist ausserdem vorgesehen, dass die unterschiedlichen Verzögerungszeitenohne gegenseitige Beeinflussung wählbar sind. Das soll jedoch nicht ausschliessen, dass auch eine feste Abhängigkeit zwischen den unterschiedlichen Verzögerungszeiten vorgesehen sein kann. In einer besonders vorteilhaften Weiterbildung der Erfindung ist vorgesehen, dass die Verzögerungszeiten programmierbar sind. Unterschiedliche Programme können dabei in bekannter Weise durch einen externen Signalgeber eingestellt werden. Eine Kopplung der beiden Verzögerungszeiten in Relation zueinander vereinfacht dabei z. B. die Programmierung.

Ein möglichst einfacher und sicherer Herzschrittmacher enthält ein Zeitverzögerungsglied, das in Abhängigkeit von den Ereignissen in der Vorkammer nach unterschiedlichen Verzögerungszeiten die Auslösung eines Stimulierungsimpulses für die Herzkammer freigibt. Dieses Verzögerungsglied lässt sich vorteilhaft möglichst einfach durch ein Zeitglied mit mindestens zwei unterschiedlichen Zeitausgängen realisieren, die in Abhängigkeit von den Ereignissen in der Vorkammer freigegeben werden. Darüber hinaus kann das Zeitglied einen Rückstelleingang aufweisen, der es durch jeden ausgelösten Herzkammerimpuls auf einen vorbestimmten Anfangswert zurücksetzt.

Zur richtigen Wahl des dem Ereignis angepassten Zeitausganges kann mindestens ein Logikglied vorgesehen sein, dem das Ausgangssignal des Detektors zuführbar ist und das den Zeitausgang nur in Abhängigkeit von einem bestimmten Detektorsignal freigibt. Als besonders einfaches Zeitglied ist ein Register vorge gesehen, das die von einem Taktgeber kommenden Impulse addiert. Dabei ist es beispielsweise möglich, durch Änderung der Taktfrequenz die unterschiedlichen Zeitverzögerungen gemeinsam im gleichen Verhältnis zu verändern. Um auch die Verzögerungszeit unabhängig voneinander verändern zu können, sind in einer vorteilhaften Weiterbildung Schaltmittel vorgesehen, durch die mindestens zwei unterschiedliche Ausgänge des Zeitgliedes für eine einzige Verzögerungszeit wählbar sind.

Im folgenden wird anhand von vier Figuren ein Ausführungsbeispiel der Erfindung näher beschrieben und erläutert.

Fig. 1 zeigt dabei ein Prinzipschaltbild eines erfindungsgemässen bifokalen inhibierten Herzschrittmachers.

Fig. 2 zeigt eine mögliche Ausführungsform des Verzögerungsgliedes gemäss der Fig. 1.

Die Fig. 3 und 4 zeigen stark vereinfachte EKG-Registrierungen für unterschiedliche Vorkammeraktivitäten.

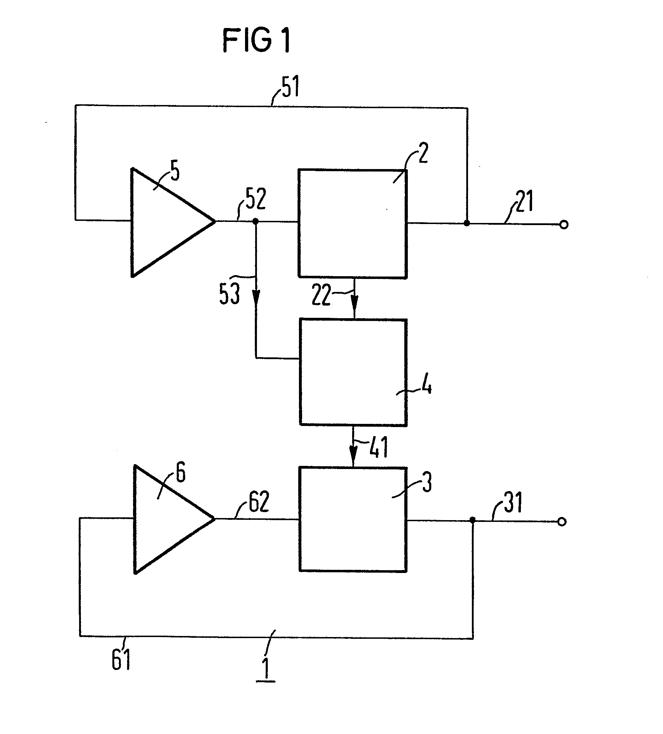

Die Fig. 1 zeigt einen Herzschrittmacher 1, der im wesentlichen aus einem Stimulierungsimpulsgenerator 2 für der Vorkammer zuzuführende Stimulierungsimpulse und einem weiteren Stimulierungsimpulsgenerator 3 für der Herzkammer zuzuführende Stimulierungsimpulse besteht. Über Leitungen 21 bzw. 31 sind die Ausgänge der beiden Generatoren 2 bzw. 3 an hier nicht dargestellte Elektroden anschliessbar. Das Ausgangssignal des Generators 2 ist weiterhin über eine Leitung 22 einem Eingang eines Verzögerungsgliedes 4 zugeführt. Weiterhin sind in bekannter Weise zwei Detektoren 5 bzw. 6 zur Registrierung der spontanen Vorkammer- bzw. Herzkammeraktivitäten vorgesehen, deren Eingänge über Leitungen 51 bzw. 61 mit den zu den entsprechenden Elektroden führenden Leitungen 21 bzw. 31 verbunden sind. Über eine Leitung 52 ist der Ausgang des Detektors 5 mit dem Generator 2 verbunden. Entsprechend ist über eine Leitung 62 der Ausgang des Detektors 6 mit dem Generator 3 verbunden. In bekannter Weise kann die Abgabe eines Stimulierungsimpulses jeweils unterdrückt oder synchronisiert werden, wenn der vorgeschaltete Detektor eine spontane Herzaktivität anzeigt. Um Störungen zu vermeiden, besitzen die Detektoren ausserdem eine gewisse Refraktärzeit, wodurch sie nach Abgabe eines Stimulierungsimpulses für eine vorbestimmte Zeitdauer gesperrt sind. Der Ausgang des Detektors 5 zur Erfassung der spontanen Aktivität der Vorkammer ist ausserdem über eine Leitung 53 mit einem weiteren Eingang des Verzögerungsgliedes 4 verbunden. Der Ausgang des Verzögerungsgliedes ist über eine Leitung 41 mit dem Generator 3 zur Erzeugung der Stimulationsimpulse für die Herzkammer verbunden.

Je nachdem, ob das Verzögerungsglied 4 ein Signal von dem Detektor 5 oder von dem Generator 2 erhält, wird nach einer unterschiedlich langen Verzögerungszeit ein Signal an den Generator 3 abgegeben, das die Auslösung eines Stimulierungsimpulses für die Herzkammer ermöglicht.

In dem Ausführungsbeispiel gemäss der Fig. 1 sind die Stimulierungsgeneratoren 2 und 3 als separate Generatoren dargestellt. Selbstverständlich kann es sich auch um einen gemeinsamen Generator handeln. Dieser kann beispielsweise jeweils ein Paar von Impulsen abgeben, wovon der erste für die Stimulierung der Vorkammer und der zweite für die Stimulierung der Herzkammer verwendet wird. Auch kann das Verzögerungsglied 4 direkt Teil des Generators sein. Die Impulserzeugung und/oder die Verzögerungszeiten können dabei oszillatorgesteuert sein. Ohne Einschränkung der Erfindung ist es ebenso möglich, dass auf den Detektor 6 zur Erfassung der spontanen Herzkammeraktivitäten verzichtet wird.

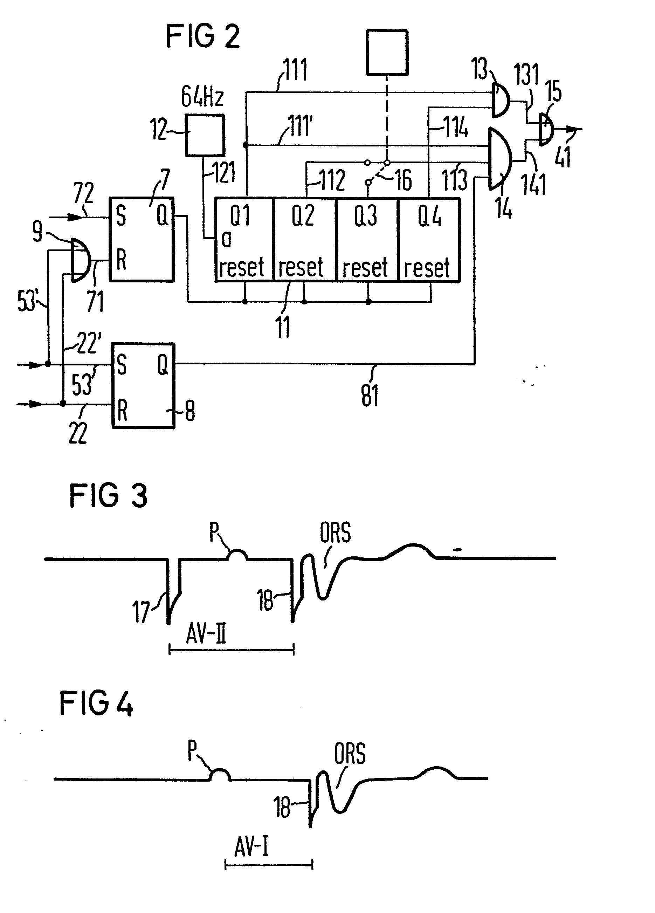

In der Fig. 2 ist eine Ausführungsform eines Verzögerungsgliedes als Blockschaltbild dargestellt. Eingangsseitig besitzt das Zeitverzögerungsglied zwei bistabile Kippstufen 7 bzw. 8 mit je einem Setzeingang S, einem Löscheingang R und einem Ausgang Q. Über eine Leitung 53 ist der Setzeingang S der Kippstufe 8 mit dem Ausgang des Detektors 5 für die Erfassung der spontanen Vorkammeraktivitäten verbunden. Der Löscheingang R dieser Kippstufe ist über eine Leitung 22 mit dem Ausgang des Impulsgenerators 2 verbunden, der Impulse zur Stimulierung der Vorkammer erzeugt. Sowohl der Detektor 5 als auch der Impulsgenerator 2 sind in die Fig. 2 nicht explizit aufgenommen. Sie entsprechen den in der Fig. 1 dargestellten Gliedern. Die über die Leitung 22 bzw. 53 kommenden Signale werden weiterhin über Leitungen 22' bzw. 53' einem Oder-Glied 9 zugeführt, dessen Ausgang über eine Leitung 71 mit dem Löscheingang R der bistabilen Kippstufe 7 verbunden ist. Dem Setzeingang S dieser Kippstufe 7 sind über eine Leitung 72 Herzkammerimpulse zuführbar.

Weiterhin enthält das Zeitverzögerungsglied ein in diesem Ausführungsbeispiel aus vier Flipflops aufgebautes Register 11, dem eingangsseitig von einem Taktgeber 12 Impulse mit einer Frequenz von beispielsweise 64 Hz über eine Leitung 121 zugeführt werden. Der Ausgang der bistabilen Kippstufe 7 ist mit sämtlichen Rücksetzeingängen der Flipflops verbunden. Die Ausgänge Q1 und Q4 des Registers 11 sind über Leitungen 111 bzw. 114 mit den Eingängen eines Und-Gliedes 13 verbunden, dessen Ausgang über eine Leitung 131 mit einem Eingang eines Oder-Gliedes 15 verbunden ist. Der Ausgang Q1 und mit Hilfe eines Schalters 16 wahlweise einer der Ausgänge Q2 bzw. Q3 sind über Leitungen 111', 112 bzw. 113 mit zwei Eingängen eines weiteren Und-Gliedes 14 verbunden, das einen zusätzlichen Eingang aufweist, der über eine Leitung 81 mit dem Ausgang Q der bistabilen Kippstufe 8 verbunden ist. Der Ausgang des Und-Gliedes 14 ist über eine Leitung 141 auf den zweiten Eingang des Oder-Gliedes 15 gegeben. Der Ausgang des Oder-Gliedes 15 steuert über eine Leitung 41, wie in Fig. 1 dargestellt, den Impulsgenerator 3 zur Erzeugung der Stimulierungsimpulse für die Herzkammer an.

Die Funktionsweise des gemäss Fig. 2 aufgebauten Verzögerungsgliedes ist folgende:
Durch jeden Herzkammerimpuls wird die bistabile Kippstufe 7 gesetzt und damit das Register 11 in einen vorgewählten Anfangszustand zurückgesetzt und dort so lange gehalten, bis entweder ein Stimulierungsimpuls für die Vorkammer oder eine spontane Vorkammeraktivität auftritt, durch die die Kippstufe 7 wieder zurückgesetzt wird. Das Register 11 wird damit freigegeben und beginnt die von dem Taktgeber 12 kommenden Taktimpulse zu zählen. Ist ein Stimulierungsimpuls für die Vorkammer abgegeben worden, so ist die bistabile Kippstufe 8 zurückgesetzt, wodurch die Leitung 81 niedriges Potential aufweist und das Und-Glied 14 gesperrt ist. Für diesen Fall erhält man ein Ausgangssignal auf der Leitung 41 zu dem Zeitpunkt, in dem die Ausgänge Q1 und Q4 des Registers beide Eins sind. Wählt man als Taktfrequenz 64 Hz, so ergibt das bei einem vierstufigen Register eine Zeitverzögerung von ca. 250 Millisekunden.

Im zweiten Fall liegt eine spontane Reaktion der Vorkammer (P-Zacke) vor, wodurch die bistabile Kippstufe 8 gesetzt ist. In diesem Fall wird bereits ein Ausgangssignal von dem Verzögerungsglied abgegeben, wenn die Ausgänge Q1 und - je nach Stellung des Schalters 16 - der Ausgang Q2 bzw. der Ausgang Q3 Eins sind. Die Verzögerungszeit für diesen Fall ist daher auf die Hälfte oder sogar ein Viertel herabgesetzt.

Ebenso vorteilhaft können auch andere Register oder Zähler eingesetzt werden, wobei die Zahl der Stufen und die verwendete Taktfrequenz den erforderlichen Verzögerungsintervallen beliebig angepasst werden kann. Weiterhin ist es auch möglich, die Taktfrequenz und damit synchron beide Verzögerungsintervalle gleichmässig zu verändern. Wird beispielsweise ein Zähler mit preset-Eingängen verwendet, so kann auf die Logikglieder verzichtet werden. Die Erfahrung hat gezeigt, dass beim Auftreten einer spontanen Vorkammerreaktion die Verzögerungszeit (AV-Zeit) in der Grössenordnung 50 bis 250 Millisekunden liegt, beim Ausbleiben einer derartigen spontanen Aktivität, d.h. also bei der notwendigen Erzeugung eines Stimulierungsimpulses für die Vorkammer, etwa bis zu 100 Millisekunden länger sein muss.

Die unterschiedliche Verzögerungszeit kann auch analog erzeugt werden. So kann z. B. ein Kondensator aufgeladen werden. Die Spannung am Kondensator wird dann in einem Komparator mit einer Vergleichsspannung verglichen, die je nach dem Ereignis in der Vorkammer zwei unterschiedliche Werte annimmt. Bei Abgleich am Komparator wird das Steuersignal für den Generator 3 abgegeben.

In der Fig. 3 ist stark vereinfacht eine EKG-Registrierung für den Fall dargestellt, dass eine spontane Reaktion der Vorkammer ausgeblieben ist. Zeitlich nacheinander wird daher zunächst ein Vorkammerimpuls 17 erzeugt, auf den die Vorkammer zeitlich verzögert mit einer P-Zacke reagiert. Nach der Verzögerungszeit AV-II wird der Stimulierungsimpuls 18 für die Herzkammer erzeugt, dem sich der QRS-Komplex anschliesst.

Die Fig. 4 zeigt eine gleiche EKG-Registrierung für den Fall einer spontanen Vorkammeraktivität. Für diesen Fall ergibt sich eine wesentlich kürzere Verzögerungszeit AV-I.

4 Figuren
10 Patentansprüche

## Patentansprüche

1. Herzschrittmacher zur bifokalen Stimulierung des Herzens mit einem Impulsgenerator, der Stimulierungsimpulse für die Vorkammer und zeitverzögert für die Herzkammer liefert und der über mindestens einen Detektor die spontanen Aktivitäten der Vorkammer erfasst und zur Steuerung des Impulsgenerators benutzt, d a d u r c h  g e k e n n z e i c h n e t , dass ein Zeitverzögerungsglied (4) vorgesehen ist, das in Abhängigkeit von den Ereignissen in der Vorkammer (spontane P-Zacke oder Stimulierungsimpuls (17)) unterschiedlich lange Verzögerungszeiten (AV-I, AV-II) erzeugt.

2. Herzschrittmacher nach Anspruch 1, d a d u r c h  g e k e n n z e i c h n e t , dass die Verzögerungszeit beim Auftreten einer spontanen P-Zacke kürzer ist als beim Auftreten eines Stimulierungsimpulses (17).

3. Herzschrittmacher nach Anspruch 1 oder 2, d a - d u r c h  g e k e n n z e i c h n e t , dass die unterschiedlichen Verzögerungszeiten (AV-I, AV-II) ohne gegenseitige Beeinflussung wählbar sind.

4. Herzschrittmacher nach Anspruch 1 oder 2, d a - d u r c h  g e k e n n z e i c h n e t , dass die unterschiedlichen Verzögerungszeiten (AV-I, AV-II) in Relation zueinander gekoppelt sind.

5. Herzschrittmacher nach einem der Ansprüche 1 bis 4, d a d u r c h  g e k e n n z e i c h n e t , dass die unterschiedlichen Verzögerungszeiten (AV-I, AV-II) programmierbar sind.

**0076363**

6. Herzschrittmacher nach einem der Ansprüche 1 bis 5, d a d u r c h   g e k e n n z e i c h n e t , dass das Verzögerungsglied (4) ein Zeitglied (11) mit mindestens zwei unterschiedlichen Zeitausgängen (Q2, Q3, Q4) aufweist, die in Abhängigkeit von den Ereignissen in der Vorkammer freigegeben werden und dass das Zeitglied (11) durch jeden ausgelösten Herzkammerimpuls auf einen vorbestimmten Anfangswert zurücksetzbar ist.

7. Herzschrittmacher nach Anspruch 1 bis 6, d a d u r c h   g e k e n n z e i c h n e t , dass mindestens zur Freigabe eines Zeitausganges (Q2, Q3) ein Logikglied (14) vorgesehen ist, dem das Ausgangssignal des Detektors (5) zuführbar ist.

8. Herzschrittmacher nach einem der Ansprüche 1 bis 7, d a d u r c h   g e k e n n z e i c h n e t , dass als Zeitglied ein Register (11) vorgesehen ist, das die von einem Taktgeber (12) kommenden Impulse zählt.

9. Herzschrittmacher nach einem der Ansprüche 1 bis 8, d a d u r c h   g e k e n n z e i c h n e t , dass Schaltmittel (16) vorgesehen sind, durch die mindestens zwei unterschiedliche Ausgänge (Q2, Q3) des Zeitgliedes (11) für eine Verzögerungszeit (AV-I) wählbar sind.

1/2

FIG 1

## FIG 2

## FIG 3

## FIG 4

Europäisches
Patentamt    **EUROPÄISCHER RECHERCHENBERICHT**

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | A 61 N 1/36 |
| A | <u>DE - A1 - 2 943 583</u> (MEDTRONIC) <br> * Seite 13, Zeilen 1-21; Seite 19, Zeile 19 - Seite 20, Zeile 18; Fig. 1,2 * <br> -- | 1 | |
| X | <u>DE - A1 - 2 701 481</u> (BIOTRONIK) <br> * Seite 1, Anspruch 1; Seite 2, Ansprüche 5-7; Seite 3, Ansprüche 8,9; Seite 7, Zeilen 1-17; Seite 8, Zeile 14 - Seite 9, Zeile 28; Fig. 1 * <br> -- | 1-4 | |
| A | <u>DE - A1 - 2 701 104</u> (MEDTRONIC) <br> * Seite 1, Ansprüche 1,2; Seite 7, Anspruch 7 - Seite 3, Anspruch 9; Seite 12, Zeilen 1-20; Fig. 1,2 * <br> -- | 1 | **RECHERCHIERTE SACHGEBIETE (Int. Cl.³)** <br><br> A 61 N |
| A | <u>DE - A1 - 2 528 817</u> (MEDTRONIC) <br> * Seite 4, Zeilen 3-21; Seite 13, Anspruch 8 - Seite 15, Anspruch 9; Fig. 1,3 * <br> -- | 1 | |
| A | <u>DE - A1 - 2 929 498</u> (MEDTRONIC) <br> * Seite 73, Zeile 15 - Seite 75, Zeile 17; Fig. 1,2 * <br> -- | 1 | |
| A | <u>DE - A - 2 255 422</u> (AMERICAN OPTICAL) <br> * Seite 1, Zeilen 5-19; Seite 9, Anspruch 1; Seite 10, Anspruch 5 * <br> -- | 1 | |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| X | Der vorliegende Recherchenbericht wurde für alle Patentansprüche erstellt. | |
|---|---|---|
| Recherchenort <br> WIEN | Abschlußdatum der Recherche <br> 21-10-1982 | Prüfer <br> NEGWER |

EPA form 1503.1   06.78

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| A | <u>DE - A - 2 255 420</u> (AMERICAN OPTICAL)<br><br>* Seite 21, Anspruch 1 *<br><br>-- | 1 | |
| A | <u>DE - A - 2 010 724</u> (AMERICAN OPTICAL)<br><br>* Seite 4, Zeile 24 - Seite 6, Zeile 10; Seite 14, Anspruch 1; Seite 16, Anspruch 10 *<br><br>---- | 1 | **RECHERCHIERTE SACHGEBIETE (Int. Cl.³)** |